# EUROPÄISCHE PATENTSCHRIFT

(11) **EP 1 655 003 B1**
(45) Veröffentlichungstag und Bekanntmachung des Hinweises auf die Patenterteilung: **04.03.2009**
(21) Anmeldenummer: 04026325.3
(22) Anmeldetag: 05.11.2004
(51) Int. Cl.: A61F 2/16

(54) **Intraokularlinse**
Intraocular lens
Lentille intraoculaire

(43) Veröffentlichungstag der Anmeldung: 10.05.2006
(73) Patentinhaber: *Acri.Tec AG Gesellschaft für ophthalmologische Produkte, 16761 Hennigsdorf (DE)
(72) Erfinder: Kammann, Jochen, Dr., 44137 Dortmund (DE); Tomalla, Mark, Dr., Mülheim/Ruhr (DE); Kreiner, Christine, Dr., 81545 München (DE)
(74) Vertreter: Eisenführ, Speiser & Partner

(56) Entgegenhaltungen:
- EP-A- 0 537 643
- EP-A- 0 563 602
- WO-A-01/62188
- US-A1- 2003 014 107
- US-A1- 2003 078 655
- US-B1- 6 428 574

## Beschreibung

Die Erfindung betrifft eine Intraokularlinse nach dem Oberbegriff des Patentanspruches 1.

Eine derartige Intraokularlinse ist aus WO 01/62188 A bekannt. Die bekannte Intraokularlinse wird zwischen Augeniris und natürlicher Augenlinse implantiert. Sie besitzt einen optischen Linsenteil und eine Haptik zur Abstützung der Linse im Bereich der Zonular-Fasern und des Ziliarmuskels. Die Haptik weist einen den optischen Linsenteil zumindest teilweise umgebenden inneren Haptikteil und einen sich daran anschließenden äußeren Haptikteil auf. An der der Augenlinse zugewandten Linsenrückseite besitzen der äußere Haptikteil eine ebene Fläche und der innere Haptikteil eine im Winkel dazu verlaufende Fläche. Der zentrale Radius der konkaven Linsenrückseite unterscheidet sich vom zentralen Radius der natürlichen Linse in ihrem nichtakkommodierten Zustand. Bei einer Ausführungsform für Patienten, die jünger als etwa 30 Jahre sind, ist der zentrale Radius der Linsenrückseite wesentlich geringer als der zentrale Radius der natürlichen Linse und beträgt weniger als 8 mm und insbesondere weniger als 7 mm. Bei Patienten, die älter als etwa 30 Jahre sind, ist der zentrale Radius der Linsenrückseite größer als der zentrale Radius der natürlichen Linse und insbesondere größer als etwa 14 mm.

Eine derartige Intraokularlinse wird bei Patienten implantiert, deren eigene natürliche Augenlinse noch voll funktionsfähig ist. Die Intraokularlinse kann sowohl bei hochmyopen (kurzsichtigen) Patienten als auch hochhyperopen (weitsichtigen) Patienten eingesetzt werden und dient ausschließlich der Korrektur der Refraktionsanomalie. Die implantierte Linse kann als Ersatz für herkömmliche Brillengläser, für auf die Kornea aufgesetzte Kontaktlinsen oder andere Korrekturverfahren, wie das Entfernen von Korneaschichten und dergleichen dienen. Bei den angesprochenen Intraokularlinsen besteht jedoch die Gefahr, dass es insbesondere im späteren Leben des Patienten zu einer vorgezogenen Trübung der natürlichen Linse (Kataraktbildung) kommen kann. Dies beruht darauf, dass durch die implantierte Linse praktisch die gesamte Vorderfläche der natürlichen Augenlinse abgedeckt ist und nur noch mangelhaft versorgt wird.

Aufgabe der Erfindung ist es, eine Intraokularlinse der eingangsgenannten Art zu schaffen, bei welcher neben einer ausreichenden Fixierung und Positionierung der Linse im Auge eine ausreichende Versorgung der natürlichen Augenlinse über die vordere Oberfläche gewährleistet bleibt.

Diese Aufgabe wird erfindungsgemäß durch die kennzeichnenden Merkmale des Patentanspruches 1 gelöst.

Bei der Erfindung ist der optische Linsenteil der Intraokularlinse an der Linsenrückseite stärker gekrümmt, als die Vorderseite der akkomondierten Augenlinse.

Hierdurch wird gewährleistet, dass zwischen der implantierten Intraokularlinse und der natürlichen Augenlinse des Patienten ständig ein Zwischenraum vorhanden ist, welcher vom Kammerwasser durchflossen wird. Damit bleibt eine ausreichende Versorgung und ein ausreichender Stoffwechsel an der Vorderseite der natürlichen Augenlinse gewährleistet.

An der Rückseite der Intraokularlinse, welche der natürlichen Augenlinse gegenüber liegt, ist der Krümmungsradius des optischen Linsenteils vorzugsweise kleiner als 5 mm bemessen und liegt insbesondere im Bereich von 4 mm bis 5 mm, beispielsweise bei 4,25 mm.

Ferner sind an der Linsenvorderseite und/oder der Linsenrückseite des optischen Linsenteils ein zentraler refraktiv wirkender Linsenbereich mit glatter Oberfläche und ein den zentralen Linsenbereich umgebender ringförmiger Linsenbereich mit ringförmigen konzentrischen Zonen vorgesehen. Der Weglängenunterschied des Strahlengangs zwischen benachbarten Zonen entspricht einer Designwellenlänge oder einem geradzahligen Vielfachen einer Designwellenlänge insbesondere im grünen Bereich des sichtbaren Lichtes. Vorzugsweise bilden der zentrale Linsenbereich und der ringförmige Linsenbereich einen gemeinsamen Fokus. Eine Intraokularlinse mit derartiger Gestaltung des optischen Bereichs ist beispielsweise in EP 0 537 643 B1 und WO 00/76426 A2 beschrieben.

Vorzugsweise haben die Zonenoberflächen an einer Linsenseite einen jeweiligen Krümmungsverlauf, welcher parallel zum Krümmungsverlauf des zentralen Linsenbereichs auf der gleichen Linsenseite ist. Die im wesentlichen parallel zur optischen Achse sich erstreckenden Stufen zwischen den einzelnen Zonen sind so bemessen, dass der Weglängenunterschied des Strahlengangs zwischen benachbarten Zonen das Ganzzahlige Vielfache der Designwellenlänge ist oder der Designwellenlänge entspricht.

Durch die beschriebene Ausgestaltung des Optikbereiches erreicht man einen äußerst flachen Linsenkörper mit geringer Linsendicke, der für die Anordnung in dem engen Zwischenraum zwischen der Augeniris und der natürlichen Augenlinse geeignet ist. Dabei bleibt ein ungestörter Fluss des Kammerwassers zur Vorderfläche der natürlichen Augenlinse gewährleistet. Ferner wird der Fluss des Kammerwassers vom Ziliarepithel in die Vorderkammer nicht behindert, da auch zwischen der Vorderseite der Intraokularlinse und der Rückseite der Augeniris ein ausreichender Raum vorhanden ist, welcher die Diffusion in die und aus den Irisgefäßen nicht behindert. Das hydrostatische Druckgefälle, welches den Fluss des Kammerwassers aus der Hinterkammer in die Vorderkammer veranlasst, wird nicht beeinträchtigt.

Eine weitere Ausgestaltung der Erfindung kann darin bestehen, dass die Haptik Einbuchtungen aufweist, welche vom äußeren Haptikrad ausgehen und sich in Richtung zur Linsenmitte hin über den gesamten äußeren Haptikteil und teilweise über den inneren Haptikteil erstrecken. Ferner können im inneren Haptikteil neben den Einbuchtungen liegende Öffnungen vorgesehen sein. Hierdurch ist gewährleistet, dass der Kammerwasserfluss auch in dem Bereich, welcher sich an das Ziliarepithel anschließt, nicht behindert wird. Die verbleibenden Haptikteile gewährleisten die ausreichende Positionierung der Intraokularlinse im Auge. Die Haptik ist ausreichend flexibel gestaltet, so dass Akkomodationsbewegungen die Positionierung des optischen Linsenteils nicht beeinflussen.

Die Intraokularlinse kann nicht nur zur Korrektur von Refraktionsanomalien bei hochmyopen und hochhyperopen Patienten zum Einsatz kommen, sondern ist auch zur Korrektur einer presbyopen Refraktionsanomalie bei alterssichtigen Patienten geeignet. Insbesondere kann hierzu die Intraokularlinse mit einer Nahaddition von +1.0 Dioptrie oder +2.0 Dioptrie in ihrem optischen Linsenteil ausgestattet sein.

Anhand der Figuren wird an einem Ausführungsbeispiel die Erfindung noch näher erläutert. Es zeigt:
- Figur 1: eine Draufsicht auf die Vorderseite eines Ausführungsbeispiels der Intraokularlinse; und
- Figur 2: eine schnittbildliche Darstellung der Intraokularlinse, entlang einer Schnittlinie II-II in Figur 1.

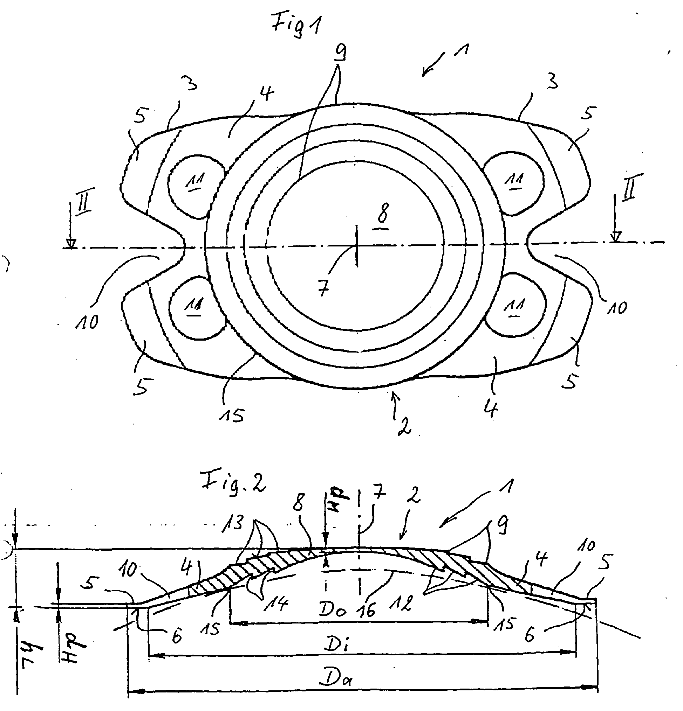

Eine in den Figuren dargestellte Intraokularlinse 1 beinhaltet einen optischen Linsenteil 2 mit kreisrundem Umfangsrand 15. Bezüglich der optischen Achse 7 ist der Körper des optischen Linsenteils 2 symmetrisch aufgebaut und beinhaltet einen zentralen Optikteil 8, der auf der Rückseite und der Vorderseite glatte Oberflächen aufweist. Die Rückseite (Unterseite in Figur 2) liegt im implantierten Zustand der Vorderseite der natürlichen Augenlinse gegenüber. Die Vorderseite (Oberseite in der Figur 2) der Linse liegt der Rückseite der Augeniris gegenüber. Der zentrale Linsenbereich 8 bildet einen refraktiven Bereich des optischen Linsenteils.

Der optische Linsenteil 2 beinhaltet ferner einen ringförmigen diffraktiven Linsenbereich 9 mit ringförmigen, um die optische Achse 7 konzentrisch angeordneten Zonen. Die einzelnen Zonen sind so gestaltet, dass sie exakt den gleichen Fokus besitzen, wie der refraktive zentrale Linsenbereich 8. Benachbarte Zonen sind durch Zonenstufen 14 voneinander getrennt. Auf der Linsenvorderseite befinden sich Zonenflächen 13 und auf der Linsenrückseite befinden sich Zonenflächen 12. Die zwischen den Zonenstufen 14 verlaufenden Zonenflächen besitzen Krümmungen, welche parallel zur jeweiligen Krümmung der Oberfläche des zentralen Linsenbereiches 8 auf der Vorderseite und der Rückseite der Intraokularlinse sind. Die Krümmungen der Zonenflächen 13 und 12 sind im wesentlichen gleich den Krümmungen des zentralen Linsenbereiches 8 an der Vorderseite und der Rückseite der Linse. Das bedeutet, die Zonenflächen 13 besitzen einen parallel Krümmungsverlauf zur Oberfläche des refraktiven zentralen Linsenbereiches 8 an der Vorderseite der Intraokularlinse 1. Die Zonenflächen 12 besitzen einen zur Oberfläche des zentralen Linsenbereichs 8 an der Rückseite der Intraokularlinse 1.

Die Höhe der im wesentlich parallel zur optischen Achse 7 sich erstreckenden Zonenstufen 14 ist so bemessen, dass der Weglängenunterschied des Strahlengangs zwischen benachbarten Zonen einer Designwellenlänge entspricht oder ein ganzzahliges Vielfaches der Designwellenlänge insbesondere im grünen Bereich des sichtbaren Spektrums ist. Durch die beschriebene diffraktive Gestaltung des ringförmigen Linsenbereiches 9 mit den konzentrisch angeordneten Zonen erreicht man eine streuzentrenarme Gestaltung der Intraokularlinse. Außerdem erreicht man eine Reduzierung der Dicke des Linsenkörpers. Die Linsendicke d_{M} im Bereich der optischen Achse 7 liegt unter 0,3 mm und kann 0,1 mm betragen.

Bei der in den Figuren dargestellten konvexkonkaven Querschnittsform der Intraokularlinse handelt es sich um eine bevorzugte Ausführungsform. Die vordere Oberfläche des optischen Linsenteils 2 kann auch plan oder konkav ausgebildet sein.

Die Oberfläche des optischen Linsenteils 2 an der Linsenrückseite ist stärker gekrümmt, als die Vorderseite der akkomodierten (d. h. auf den Nahbereich eingestellten) natürlichen Augenlinse. Insbesondere ist der Krümmungsradius der Oberfläche des optischen Linsenteils 2 an der Linsenrückseite kleiner als 5 mm und kann 4,25 mm betragen. Auf diese Weise ist gewährleistet, dass auch bei akkomodiertem Auge zwischen der Linsenrückseite der Intraokularlinse 1 und der Vorderfläche der akkomodierten natürlichen Augenlinse ein ausreichender Abstand für die Benetzung der natürlichen Augenlinse durch das Kammerwasser vorhanden ist.

Ein kreisrunder Umfangsrand 15 des optischen Linsenteils 2 liegt an der Linsenrückseite auf einer fiktiven Kugeloberfläche, deren Radius 10 mm bis 10,2 mm ist, und deren Mittelpunkt auf der optischen Linsenachse 7 liegt. Die übrigen Teile der Linsenrückfläche liegen außerhalb der Kugeloberfläche 16, wie aus der Fig. 2 zu ersehen ist.

Ferner beinhaltet die dargestellte Intraokularlinse 1 eine Haptik 3, welche zur Positionierung und Abstützung der Intraokularlinse im Auge, insbesondere im Bereich der Zonulafasern und des Ziliarkörpers, insbesondere der Ziliarfortsätze dient. Die Haptik 3 besitzt einen inneren Haptikteil 4, der sich unmittelbar an den optischen Linsenteil 2 anschließt. Ferner besitzt die Haptik 3 einen äußeren Haptikteil 5, der sich radial nach außen an den inneren Haptikteil anschließt. Beim dargestellten Ausführungsbeispiel sind bezüglich der optischen Linsenachse 7 diametral liegende Haptikbereiche vorgesehen. Die beiden Haptikbereiche besitzen etwa V-förmige Einbuchtungen 10 mit abgerundeten Rändern und abgerundeten Einbuchtungsböden. Die Einbuchtungen 10 erstrecken sich in Richtung zur optischen Achse 7 hin über den gesamten äußeren Haptikteil 5 und teilweise in den inneren Haptikteil 4, wie insbesondere als Figur 1 zu ersehen ist. Es entstehen hierdurch Haptikstützfüße, mit denen die Intraokularlinse 1 im Auge positioniert wird. Beim Ausführungsbeispiel sind vier Haptikfüsse vorgesehen. Die inneren Haptikteile 4 besitzen beidseits einer Mittelinie Öffnungen 11. Die Öffnungen 11 können teilweise durch den äußeren Umfangsrand 15 des optischen Linsenteils 2 begrenzt sein. Sowohl durch die Öffnungen 11 als auch durch die Einbuchtungen 10 werden Kanäle für das Kammerwasser geschaffen, so dass dieses vom Ziliarepithel ausgehend zwischen der Augeniris und der natürlichen Augenlinse in die Vorderkammer fließen kann. Akkomodationsbewegungen des Auges wirken sich auf die Positionierung des optischen Linsenteils 2 aufgrund der Flexibilität der Haptikteile nicht aus.

Die zwischen den Zonenflächen 12 und 13 liegenden Zonenstufen erstrecken sich im wesentlichen parallel zur optische Achse 7. Sie begrenzen die einzelnen Zonen im ringförmigen Linsenbereich 9. Beim dargestellten Ausführungsbeispiel besitzt der ringförmige Linsenbereich 9 drei Zonen mit den Zonenflächen 12 und 13 und den dazwischen liegenden Zonenstufen 14.

Der Durchmesser Do des optischen Linsenteils 2 beträgt 6 mm. Der Durchmesser Di des inneren Haptikteils 4 beträgt 10 mm. Der Gesamtdurchmesser Da der Intraokularlinse 1 kann zwischen 11 mm und 13 mm je nach Physiologie des Auges, in welches die Intraokularlinse implantiert werden soll, liegen.

Die Dicke d_{H} des äußeren Haptikteils 5 beträgt beim dargestellten Ausführungsbeispiel 0,1 mm und kann auch größer sein. Die gesamte Höhe h_{L} der Intraokularlinse 1 beträgt 1,44 mm.

Der äußere Haptikbereich 5 verläuft gegenüber der optischen Achse 7 im wesentlichen in einem rechten Winkel und besitzt an seiner Rückseite ebene Oberflächen 6. Die inneren Haptikteile 4 verlaufen schräg gegenüber den äußeren Haptikteilen 5, so dass der Linsenkörper der Intraokularlinse 1 eine domartig gewölbte Grundform, wie im Querschnitt der Figur 2 zu ersehen ist, aufweist. Die domartig gewölbte Grundform der Linse befindet sich außerhalb der Kugeloberfläche 16, deren Mittelpunkt auf der optischen Achse 7 liegt. Lediglich im Bereich des äußeren Umfangsrandes 15 an der Rückseite des optischen Linsenteils 2 kann eine Berührung mit dieser fiktiven Kugelfläche 16 vorhanden sein. Der Umfangsrand 15 an der Linsenrückseite kann jedoch auch um einen geringen Betrag außerhalb der fiktiven Kugelfläche 16 liegen. Die innenliegende Haptik 4 besitzt an der Linsenrückseite eine schräge Fläche, welche im Winkel zur Tangente an die Kugeloberfläche 16 beim Umfangsrand 15 an der Linsenrückfläche verläuft. Wie oben schon erläutert, liegt die Fläche an der Rückseite der innenliegenden Haptik 4 außerhalb der fiktiven Kugeloberfläche 16. Die an der Rückseite des inneren Haptikteils 4 befindliche Fläche weist gegenüber der ebenen Oberfläche 6 an der Rückseite des äußeren Haptikbereiches 5 ebenfalls einen Neigungswinkel auf, welcher in der Größenordnung von 14° oder geringfügig darüber oder darunter liegen kann.

### [Bezugszeichenliste]

- 1: Intraokularlinse
- 2: optischer Linsenteil
- 3: Haptik
- 4: innerer Haptikteil
- 5: äußerer Haptikteil
- 6: ebene Fläche am äußeren Haptikteil
- 7: optische Achse
- 8: zentraler Linsenbereich
- 9: ringförmiger Linsenbereich
- 10: Einbuchungen in der Haptik
- 11: Öffnungen
- 12, 13: Zonenoberflächen
- 14: Zonenstufen
- 15: Umfangsrand des optischen Linsenteils
- 16: Kugelfläche

## Patentansprüche

1. Intraokularlinse, welche zwischen Augeniris und natürlicher Augenlinse zu implantieren ist, mit einem optischen Linsenteil (1) und einer Haptik (3), die eine den optischen Linsenteil (2) zumindest teilweise umgebenden Haptikteil (4) und einen sich daran anschließenden äußeren Haptikteil (5) aufweist, wobei zumindest an der der Augenlinse zugewandten Linsenrückseite der äußere Haptikteil (5) eine ebene Fläche und der innere Haptikteil (4) eine im Winkel dazu verlaufende Fläche aufweisen, wobei der optische Linsenteil (2) an der Linsenrückseite stärker gekrümmt ist als die Vorderseite der akkommodierten Augenlinse,
**dadurch gekennzeichnet, dass** der kreisförmige Umfangsrand des optischen Linsenteils (2) auf einer fiktiven Kugelfläche (16) mit einem Mittelpunkt auf der optischen Linsenachse (7) und einem Radius von 10 mm bis 10,2 mm liegt und dass die übrigen Teile der Linsenrückseite außerhalb der fiktiven Kugelfläche (16) liegen.

2. Intraokularlinse nach Anspruch 1,
**dadurch gekennzeichnet, dass** der Krümmungsradius des optischen Linsenteils (2) an der Linsenrückseite kleiner als 5 mm ist

3. Intraokularlinse nach Anspruch 1,
**dadurch gekennzeichnet, dass** der Krümmungsradius des optischen Linsenteils (2) an der Linsenrückseite 4,25 mm beträgt.

## Claims

1. An intraocular lens which is to be implanted between the iris of the eye and the natural lens of the eye, comprising an optical lens portion (1) and a haptic (3) having a haptic portion (4) at least partially surrounding the optical lens portion (2) and an outer haptic portion (5) adjoining the haptic portion (4), wherein at least at the rear side of the lens that is towards the lens of the eye the outer haptic portion (5) has a flat surface and the inner haptic portion (4) has a surface extending at an angle thereto, wherein the optical lens portion (2) is more greatly curved at the rear side of the lens than the front side of the accommodated lens of the eye,
**characterised in that** the circular peripheral edge of the optical lens portion (2) is on a notional spherical surface (16) with a centre point on the optical lens axis (7) and of a radius of 10 mm to 10.2 mm and that the other parts of the rear side of the lens are outside the notional spherical surface (16).

2. An intraocular lens according to claim 1 **characterised in that** the radius of curvature of the optical lens portion (2) at the rear side of the lens is less than 5 mm.

3. An intraocular lens according to claim 1 **characterised in that** the radius of curvature of the optical lens portion (2) at the rear side of the lens is 4.25 mm.

## Revendications

1. Lentille intraoculaire à implanter entre l'iris et le cristallin naturel, comprenant une partie (1) optique de lentille et une haptique (3) qui a une partie (4) d'haptique entourant, au moins en partie, la partie (2) optique de lentille et une partie (5) haptique extérieure s'y rattachant, dans laquelle, au moins du côté arrière de la lentille tournée vers le cristallin, la partie (5) haptique extérieure a une surface plane et la surface (4) haptique intérieure a une surface faisant un angle avec la surface plane, la partie (2) optique de lentille étant plus courbée du côté arrière de la lentille que la partie avant du cristallin d'accommodation,
**caractérisée en ce que** le bord périphérique circulaire de la partie (2) optique de lentille se trouve sur une surface (16) sphérique fictive ayant un centre sur l'axe (7) optique de la lentille et un rayon de 10 mm à 10,2 mm et **en ce que** les autres parties du côté arrière de la lentille sont à l'extérieur de la surface (16) sphérique fictive.

2. Lentille intraoculaire suivant la revendication 1, **caractérisée en ce que** le rayon de courbure de la partie (2) optique de lentille sur le côté arrière de la lentille est plus petit que 5 mm.

3. Lentille intraoculaire suivant la revendication 1, **caractérisée en ce que** le rayon de courbure de la partie (2) optique de lentille sur le côté arrière de la lentille est égal à 4,25 mm.
